(19) Europäisches Patentamt
European Patent Office
Office européen des brevets

(11) **EP 3 950 303 A1**

(12) **EUROPEAN PATENT APPLICATION**
published in accordance with Art. 153(4) EPC

(43) Date of publication:
**09.02.2022 Bulletin 2022/06**

(21) Application number: **20779484.3**

(22) Date of filing: **25.03.2020**

(51) International Patent Classification (IPC):
**B32B 5/26** (2006.01)    **D04H 3/007** (2012.01)
**D04H 3/016** (2012.01)    **D04H 3/147** (2012.01)
**D04H 3/16** (2006.01)    **A61F 13/51** (2006.01)

(52) Cooperative Patent Classification (CPC):
**A61F 13/51; B32B 5/26; D04H 3/007; D04H 3/016;
D04H 3/147; D04H 3/16**

(86) International application number:
**PCT/JP2020/013446**

(87) International publication number:
**WO 2020/196663 (01.10.2020 Gazette 2020/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.03.2019 JP 2019061595**

(71) Applicant: **Mitsui Chemicals, Inc.
Tokyo 105-7122 (JP)**

(72) Inventors:
• **SAITA, Shohei**
  **Sodegaura-shi, Chiba 299-0265 (JP)**
• **OTA, Kosuke**
  **Sodegaura-shi, Chiba 299-0265 (JP)**
• **ICHIKAWA, Taiichiro**
  **Sodegaura-shi, Chiba 299-0265 (JP)**

(74) Representative: **Hoffmann Eitle
Patent- und Rechtsanwälte PartmbB
Arabellastraße 30
81925 München (DE)**

(54) **NONWOVEN LAMINATED BODY AND SANITARY PRODUCT**

(57) A nonwoven laminated body and a sanitary product, each including a nonwoven fabric layer A including a crimped composite fiber and a nonwoven fabric layer B including an extra-fine fiber having an average fiber diameter of less than 1.35 $\mu$m, in which a ratio of a basis weight of the nonwoven fabric layer B with respect to a total basis weight of the nonwoven laminated body is from 2.0% to 8.0%.

FIG.1

**Description**

Technical Field

The present disclosure relates to a nonwoven laminated body and a sanitary product.

Background Art

Nonwoven fabrics using polyolefins (for example, polypropylene) are demanded to be further improved in characteristics, while are excellent in air permeability and the like and thus are utilized as sanitary materials for paper diapers, hygiene products, and the like. For example, nonwoven fabrics are demanded which use polypropylene further enhanced in flexibility, bulkiness, and mechanical strength.

Various methods of crimping fibers using polyolefins included in nonwoven fabrics are proposed as methods of obtaining nonwoven fabrics excellent in flexibility, bulkiness, and the like.

For example, Patent Literature 1 describes a more highly crimped composite fiber which is a composite fiber having an eccentric core/sheath structure with two propylene-based polymers different in melting point, in which a higher-melting point propylene-based polymer higher in Mz/Mw than a lower melting point propylene-based polymer for use in a sheath part is used in a core part.

Patent Literature 2 and Patent Literature 3 each describe a composite in which a melt blown fiber is stacked on an intermediate layer of a laminated body of a crimped composite fiber.

Patent Literature 1: WO 2011-129211
Patent Literature 2: US Patent Application Laid-Open No. 2017/0335498
Patent Literature 3: WO 2017/198336

SUMMARY OF INVENTION

Technical Problem

It is considered that the composite fiber described in Patent Literature 1 has a concern about an increase in neck-in during processing and is problematic in terms of stable production during high-speed formation.

The "neck-in" in the disclosure means, for example, a phenomenon in which the length of a nonwoven fabric in the CD direction (direction perpendicular to the fiber flow direction) becomes shorter when the non-woven fabric is pulled in the MD direction (fiber flow direction).

It is considered that the composites described in Patent Literature 2 and Patent Literature 3 are each large in fiber diameter and basis weight in a melt blown layer introduced as an intermediate layer, and relatively high in flexural rigidity. It is thus presumed that a nonwoven laminated body by itself can be inferior in flexibility (for example, cushioning properties and draping properties) and neck-in resistance which are important particularly in a sanitary product.

An object to be achieved by an embodiment of the disclosure is to provide a nonwoven laminated body and a sanitary product which are each made low in flexural rigidity and thus each exhibit favorable flexibility and which are each excellent in neck-in resistance.

Solution to Problem

Specific means for solving the above described problems are as follows.

<1> A nonwoven laminated body comprising:

a nonwoven fabric layer A comprising a crimped composite fiber; and
a nonwoven fabric layer B comprising an extra-fine fiber having an average fiber diameter of less than 1.35 $\mu$m, wherein a ratio of a basis weight of the nonwoven fabric layer B with respect to a total basis weight of the nonwoven laminated body is from 2.0% to 8.0%.

<2> The nonwoven laminated body according to <1>, wherein the extra-fine fiber has an average fiber diameter of 1.00 $\mu$m or more and less than 1.35 $\mu$m.

<3> The nonwoven laminated body according to <1> or <2>, wherein the ratio of the basis weight of the nonwoven fabric layer B with respect to the total basis weight of the nonwoven laminated body is from 5.0% to 8.0%.

<4> The nonwoven laminated body according to any one of <1> to <3>, wherein:

the nonwoven fabric layer A is a spunbond nonwoven fabric layer, and
the nonwoven fabric layer B is a melt blown nonwoven fabric layer.

<5> The nonwoven laminated body according to any one of <1> to <4>, wherein:

the crimped composite fiber has at least two regions including a part (a) formed of an olefin-based polymer (A) and a part (b) formed of an olefin-based polymer (B),
a mass ratio [(a):(b)] between the part (a) and the part (b) is from 10:90 to 60:40, and
at least one of a condition in which a melting point of the olefin-based polymer (A) is higher than a melting point of the olefin-based polymer (B) or a condition in which Mz/Mw of the olefin-based polymer (A) is larger than the Mz/Mw of the olefin-based polymer (B) is satisfied.

<6> The nonwoven laminated body according to <5>, wherein the olefin-based polymer (A) and the olefin-based polymer (B) are each a propylene-based polymer.
<7> The nonwoven laminated body according to <5> or <6>, wherein the crimped composite fiber has an eccentric core/sheath structure in which the part (a) is a core part (a') and the part (b) is a sheath part (b').
<8> The nonwoven laminated body according to any one of <1> to <7>, wherein the nonwoven laminated body is heat-fused by embossing.
<9> The nonwoven laminated body according to <8>, wherein an embossing area ratio by the embossing is from 5% to 20%.
<10> A sanitary product comprising the nonwoven laminated body according to any one of <1> to <9>.

Advantageous Effect of Invention

According to an embodiment of the disclosure, a nonwoven laminated body and a sanitary product can be provided which are each made low in flexural rigidity and thus each exhibit favorable flexibility and which are each excellent in neck-in resistance.

BRIEF DESCRIPTION OF DRAWINGS

Figure 1 is a perspective view illustrating one example of a crimped composite fiber in the disclosure.
Figure 2 is a view describing a flexibility test method of a nonwoven fabric.
Figure 3 is a cross-sectional view illustrating one example of the crimped composite fiber in the disclosure.
Figure 4 is a cross-sectional view illustrating one example of the crimped composite fiber in the disclosure.
Figure 5 is a cross-sectional view illustrating one example of the crimped composite fiber in the disclosure.
Figure 6 is a cross-sectional view illustrating one example of the crimped composite fiber in the disclosure.
Figure 7 is a cross-sectional view illustrating one example of the crimped composite fiber in the disclosure.
Figure 8 is a cross-sectional view illustrating one example of the crimped composite fiber in the disclosure.
Figure 9 is a schematic view illustrating one example of a production apparatus of a spunbond nonwoven fabric in the disclosure.

DESCRIPTION OF EMBODIMENTS

Hereinafter, the nonwoven laminated body and the sanitary product of the disclosure will be described in detail, but the disclosure is not intended to be limited to the following embodiments and can be appropriately modified and carried out within the scope of the object of the disclosure.
A numerical value range represented by "(from) ... to ..." in the disclosure means that the range encompasses respective numerical values described before and after "to" as a lower limit and an upper limit, respectively. In a numerical value range described stepwise in the disclosure, an upper limit value or a lower limit value described with respect to a certain numerical value range may be replaced with an upper limit value or a lower limit value of other numerical value range described stepwise. In a numerical value range described stepwise in the disclosure, an upper limit value or a lower limit value described with respect to a certain numerical value range may be replaced with any value indicated in Examples.
The term "step" in the disclosure encompasses not only an independent step, but also a step that can achieve an object of the relevant step even in the case of being not clearly distinguished from other steps.
The amount of each component in a composition in the disclosure means, in a case in which a plurality of substances corresponding to such each component are present in the composition, the total amount of the plurality of substances present in the composition, unless particularly noted.

In the disclosure, the "MD" (Machine Direction) direction refers to a direction of movement of a nonwoven web in a nonwoven fabric production apparatus. The "CD" (Cross Direction) direction refers to a direction which is perpendicular to the MD direction and parallel to a main surface (a surface perpendicular to the thickness direction of a nonwoven fabric).

In a case in which the amount of each component in a composition is mentioned in the disclosure, the amount means, in a case in which a plurality of substances corresponding to such each component are present in the composition, the total amount of the plurality of substances present in the composition, unless particularly noted.

The term "layer" in the disclosure encompasses, in the case of observation of a region in which the relevant layer is present, not only any layer formed in the entire region, but also any layer formed on only a portion of the region.

«Nonwoven Laminated Body»

The nonwoven laminated body of the disclosure includes a nonwoven fabric layer A including a crimped composite fiber and a nonwoven fabric layer B including an extra-fine fiber having an average fiber diameter of less than 1.35 $\mu$m, in which the ratio of the basis weight of the nonwoven fabric layer B with respect to the total basis weight of the nonwoven laminated body is from 2.0% to 8.0%.

A laminated body using only a nonwoven fabric layer including a crimped composite fiber is large in width of shrinkage in neck-in and tends to be inferior in neck-in resistance.

A nonwoven fabric layer B including an extra-fine fiber can cause an increase in flexural rigidity and deterioration in flexibility, while allows for an enhancement in neck-in resistance.

The nonwoven laminated body of the disclosure has the above configuration, therefore is made low in flexural rigidity and thus exhibits favorable flexibility, and is excellent in neck-in resistance.

The extra-fine fiber is a fiber finer than the crimped composite fiber. Thus, the extra-fine fiber is further uniformly dispersed in the nonwoven laminated body, as compared with the crimped composite fiber.

The extra-fine fiber can also contribute to retention of the shape of the nonwoven laminated body, and thus it is presumed that the nonwoven laminated body includes the extra-fine fiber, thereby allowing for suppression of shrinkage (namely, neck-in phenomenon) of the nonwoven laminated body in vertical and horizontal directions in the case of pulling of the nonwoven laminated body in horizontal and vertical directions.

The "flexibility" in the disclosure means flexibility of the nonwoven laminated body by itself due to flexural rigidity made low and excellent draping properties.

<Nonwoven Fabric Layer A Including Crimped Composite Fiber>

The nonwoven laminated body of the disclosure includes a nonwoven fabric layer A including a crimped composite fiber.

The nonwoven laminated body of the disclosure includes the nonwoven fabric layer A including a crimped composite fiber, whereby the nonwoven laminated body can be reduced in flexural rigidity and enhanced in flexibility.

The crimped composite fiber is preferably a spunbond fiber produced by a spunbond method from the viewpoint that the nonwoven laminated body is reduced in flexural rigidity and thus is enhanced in flexibility.

The nonwoven fabric layer A may include any of various known nonwoven fabrics such as a spunbond nonwoven fabric, a card-type air-through nonwoven fabric, an air laid nonwoven fabric, a needle-punched spunbond nonwoven fabric, a wet nonwoven fabric, a dry nonwoven fabric, a dry pulp nonwoven fabric, a flash spun nonwoven fabric, and an open nonwoven fabric, other than the crimped composite fiber, and preferably includes only the crimped composite fiber.

The crimped composite fiber can be formed as, for example, a composite fiber having a cross section shape that can be crimped.

The crimped composite fiber can be formed from a plurality of polymers according to a spunbond method, and examples of such each polymer include an olefin-based polymer, a polyester-based polymer, and nylon.

In particular, such each polymer is preferably an olefin-based polymer from the viewpoint of flexibility, strength, and water resistance of the nonwoven laminated body by itself.

The crimped composite fiber in the disclosure may have, for example, at least two regions of a part (a) formed of a specified polymer and a part (b) formed of other polymer different from the specified polymer.

The crimped composite fiber in the nonwoven laminated body of the disclosure preferably has at least two regions of a part (a) formed of an olefin-based polymer (A) and a part (b) formed of an olefin-based polymer (B).

(Olefin-Based Polymer (A))

Examples of the olefin-based polymer (A) include an ethylene-based polymer and a propylene-based polymer. In particular, the olefin-based polymer (A) is preferably a propylene-based polymer.

The olefin-based polymer (A) may include one polymer, or may include two or more polymers, namely, may be a mixture of two or more polymers.

The ethylene-based polymer is a polymer mainly containing a structure unit derived from ethylene, and the concept thereof encompasses an ethylene homopolymer and a copolymer of ethylene and any $\alpha$-olefin other than ethylene.

For example, the ethylene-based polymer may be any of an ethylene homopolymer and a copolymer (ethylene/$\alpha$-olefin random copolymer) of ethylene and any $\alpha$-olefin other than ethylene.

The ethylene/$\alpha$-olefin random copolymer is preferably, for example, a random copolymer of ethylene and one or more $\alpha$-olefins other than ethylene, each having from 2 to 10 carbon atoms.

Specific examples of such any $\alpha$-olefin to be copolymerized with ethylene include propylene, 1-butene, 1-pentene, 1-hexene, 1-octene, 1-decene, 3-methyl-1-butene, 3-methyl-1-pentene, 3-ethyl-1-pentene, 4-methyl-1-pentene, and 4-methyl-1-hexene, from the viewpoint of excellent flexibility.

Examples of the ethylene/$\alpha$-olefin random copolymer include an ethylene/propylene random copolymer and an ethylene/1-butene random copolymer.

The ethylene-based polymer may include two or more different ethylene-based polymers.

The propylene-based polymer for use as the olefin-based polymer (A) is a polymer mainly containing a structure unit derived from propylene, and the concept thereof encompasses a propylene homopolymer and a copolymer (propylene/$\alpha$-olefin random copolymer) of propylene and any $\alpha$-olefin other than propylene.

For example, the propylene-based polymer may be a propylene homopolymer, may be a copolymer of propylene and any $\alpha$-olefin other than propylene, or may include both a propylene homopolymer and a copolymer of propylene and any $\alpha$-olefin other than propylene.

The propylene/a-olefin random copolymer is preferably a random copolymer of propylene and one or more $\alpha$-olefins other than propylene, each having from 2 to 10 carbon atoms.

Specific examples of such any $\alpha$-olefin to be copolymerized with propylene preferably include ethylene, 1-butene, 1-pentene, 1-hexene, 1-octene, 1-decene, 3-methyl-1-butene, 3-methyl-1-pentene, 3-ethyl-1-pentene, 4-methyl-1-pentene, and 4-methyl-1-hexene, from the viewpoint of excellent flexibility.

Examples of the propylene/a-olefin random copolymer include a propylene/ethylene random copolymer and a propylene/ethylene/1-butene random copolymer.

The content of the $\alpha$-olefin in the propylene/a-olefin random copolymer is not particularly limited, and is preferably 10% by mol or less, more preferably 5% by mol or less, still more preferably 2% by mol or less, particularly preferably 1% by mol or less.

The propylene-based polymer may include two or more different propylene-based polymers, or may include a propylene-based polymer and an ethylene-based polymer.

In particular, the olefin-based polymer (A) is preferably a homopolymer of propylene from the viewpoint of excellent mechanical strength.

The melt flow rate (MFR) (ASTM D-1238, 230°C, load 2,160 g) of the olefin-based polymer (A) is preferably from 20 g/10 min to 100 g/10 min, more preferably from 30 g/10 min to 80 g/10 min, from the viewpoint of spinnability.

In this regard, the MFR is 100 g/10 min or less, whereby the resulting nonwoven laminated body can be enhanced in tensile strength and the like.

The olefin-based polymer (A) in the disclosure is preferably higher in melting point than the olefin-based polymer (B), and the difference in melting point therebetween is preferably more than 10°C, more preferably in a range of from 12°C to 40°C.

The difference in melting point between the olefin-based polymer (A) and the olefin-based polymer (B) is larger, whereby the crimped composite fiber can be obtained from a composite fiber more excellent in crimpability.

The melting point of the olefin-based polymer (A) in the disclosure is preferably 155°C or more, more preferably from 157°C to 165°C.

The melting point of the olefin-based polymer (A) is 155°C or more, whereby the difference between the melting point of the olefin-based polymer (A) and the melting point of the olefin-based polymer (B) is more easily more than 10°C and thus the crimped composite fiber can be obtained from a composite fiber excellent in crimpability.

The ratio [Mz/Mw (A)] between the Z average molecular weight (Mz) and the weight average molecular weight (Mw) of the olefin-based polymer (A) in the disclosure is preferably 2.0 or more from the viewpoint that favorable crimpability is expressed, and the Mz/Mw (A) is preferably 4.5 or less from the viewpoint of spinnability.

The Mz/Mw (A) of the olefin-based polymer (A) is more preferably from 2.1 to 4.5, still more preferably from 2.1 to 3.0 from the same viewpoints.

The olefin-based polymer (A) in the disclosure preferably has a Mw of from 150000 to 250000 and preferably has a Mz of from 300000 to 600000.

The ratio [Mw/Mn (A)] between the weight average molecular weight (Mw) and the number average molecular weight (Mn) of the olefin-based polymer (A) in the disclosure, defined as a molecular weight distribution, is preferably from 2.0 to 4.0, more preferably from 2.2 to 3.5.

The Mz, Mw, Mn, Mz/Mw (A), and Mw/Mn (A) of the olefin-based polymer (A) in the disclosure can be measured with GPC (gel permeation chromatography) according to methods described below.

The olefin-based polymer (A) in the disclosure is usually obtained by homopolymerization of, for example, propylene, or copolymerization of, for example, propylene and a small amount of any $\alpha$-olefin, according to slurry polymerization, gas-phase polymerization, or bulk polymerization by use of a so-called Ziegler-Natta catalyst as a combination of a titanium-containing solid transition metal component and an organometallic component, or a metallocene catalyst including a compound of any transition metal belonging to the Groups 4 to 6 in the periodic table, the compound having at least one cyclopentadiene backbone, and a co-catalyst component. The olefin-based polymer can also be here produced by multistage polymerization or direct polymerization of propylene-based polymers different in MFR, particularly, a propylene-based polymer and a propylene-based polymer lower in MFR than the propylene-based polymer so that the Mz, Mw, and Mz/Mw are within the above ranges.

The Mw/Mn (A) and Mz/Mw (A) of the olefin-based polymer (A) can be adjusted by, for example, a method of adjustment with a specified catalyst in polymerization conditions, a method of adjustment by polymer decomposition with peroxide or the like, or a method of adjustment by mixing two or more polymers different in molecular weight from each other.

The olefin-based polymer (A) in the disclosure, here used, can also be a commercially available product, for example, a propylene-based polymer manufactured and sold under the trade name of NOVATEC PP SA06A from Japan Polypropylene Corporation.

The olefin-based polymer (A) in the disclosure can be, if necessary, compounded with any additive commonly used, such as an antioxidant, a weathering stabilizer, an antistatic agent, an antifogging agent, an anti-blocking agent, a lubricant, a nucleating agent, or a pigment, or any other polymer, as long as the object of the disclosure is not impaired.

(Olefin-Based Polymer (B))

Examples of the olefin-based polymer (B) include an ethylene-based polymer and a propylene-based polymer.

Specifics of the ethylene-based polymer in the olefin-based polymer (B) are the same as specifics of the ethylene-based polymer described in the section regarding the olefin-based polymer (A), and much the same is also true for preferable aspects.

Specifics of the propylene-based polymer in the olefin-based polymer (B) are the same as specifics of the propylene-based polymer described in the section regarding the olefin-based polymer (A), and much the same is also true for preferable aspects.

The following aspect with respect to the propylene-based polymer in the olefin-based polymer (B) is also preferable.

The propylene-based polymer for use as the olefin-based polymer (B) may be a homopolymer of propylene, or may be a copolymer of any propylene listed in the section of the olefin-based polymer (A) and a small amount of one or more $\alpha$-olefins (herein, except for propylene).

In particular, the propylene-based polymer for use as the olefin-based polymer (B) is more preferably a copolymer of propylene and a small amount of one or more $\alpha$-olefins, and in particular, still more preferably a propylene/a-olefin random copolymer such as a propylene/ethylene random copolymer or a propylene/ethylene/1-butene random copolymer, from the viewpoint of neck-in resistance and flexibility which are well-balanced.

The melt flow rate (MFR) (ASTM D-1238, 230°C, load 2,160 g) of the olefin-based polymer (B) is preferably in the same range as that of the MFR of the olefin-based polymer (A), from the same viewpoint.

The melting point of the olefin-based polymer (B) is preferably in a range of from 120°C to 155°C, more preferably in a range of from 125°C to 150°C.

The melting point of the olefin-based polymer (B) is 120°C or more, whereby heat resistance can be favorably retained.

The ratio [Mz/Mw (B)] between the Z average molecular weight (Mz) and the weight average molecular weight (Mw) of the olefin-based polymer (B) is preferably 2.5 or less, more preferably 2.3 or less.

The olefin-based polymer (B) preferably has a Mw in a range of from 150000 to 250000, and preferably has a Mz in a range of from 300000 to 600000.

The ratio [Mw/Mn (B)] between the weight average molecular weight (Mw) and the number average molecular weight (Mn) of the olefin-based polymer (B), defined as a molecular weight distribution, is preferably in a range of from 2.0 to 4.0, more preferably in a range of from 2.2 to 3.5.

The Mz, Mw, Mn, Mz/Mw (B), and Mw/Mn (B) of the olefin-based polymer (B) in the disclosure can be measured with GPC (gel permeation chromatography) according to methods described below.

The olefin-based polymer (B) in the disclosure can be produced by the same polymerization method as in the olefin-based polymer (A), and, in a case in which, for example, a propylene/a-olefin random copolymer is as the olefin-based polymer (B), the olefin-based polymer may also be produced by multistage polymerization or direct polymerization of small amounts of propylene/a-olefin random copolymers different in MFR so that the Mz, Mw, and the Mz/Mw are within the above ranges.

Examples of the method of adjusting the Mw/Mn (B) and Mz/Mw (B) of the olefin-based polymer (B) include a method of adjustment with a specified catalyst in polymerization conditions, a method of adjustment by polymer decomposition with peroxide or the like, and a method of adjustment by mixing two or more polymers different in molecular weight from each other.

The olefin-based polymer (B) here used can also be a commercially available product, for example, a propylene-based polymer manufactured and sold under the trade name of PRIME POLYPRO S119 from Prime Polymer Co., Ltd.

The olefin-based polymer (B) in the disclosure can be, if necessary, compounded with any additive commonly used, such as an antioxidant, a weathering stabilizer, an antistatic agent, an antifogging agent, an anti-blocking agent, a lubricant, a nucleating agent, or a pigment, or any other polymer, as long as the object of the disclosure is not impaired.

The olefin-based polymer (A) and the olefin-based polymer (B) are preferably each a propylene-based polymer, and, preferably, the olefin-based polymer (A) is a propylene homopolymer and the olefin-based polymer (B) is a propylene/a-olefin random copolymer, from the viewpoint that the resulting nonwoven laminated body has mechanical strength, neck-in resistance, and flexibility in a well-balanced manner.

It is preferable in the disclosure to satisfy at least one of a condition in which the melting point of the olefin-based polymer (A) is higher than the melting point of the olefin-based polymer (B) or a condition in which the Mz/Mw of the olefin-based polymer (A) is larger than the Mz/Mw of the olefin-based polymer (B). Thus, the crimped composite fiber can be improved in crimpability.

The melting point (Tm), Mz/Mw, and MFR with respect to each of the olefin-based polymer (A) and the olefin-based polymer (B) more preferably satisfy the following relationships.

<ΔTm>

The melting point of the olefin-based polymer (A) is preferably higher than the melting point of the olefin-based polymer (B) in the disclosure.

Thus, the crimped composite fiber can be obtained from a composite fiber more excellent in crimpability.

The difference between the melting point of the olefin-based polymer (A) forming the part (a) and the melting point of the olefin-based polymer (B) forming the part (b) in the disclosure is preferably more than 10°C, more preferably from 12°C to 40°C, from the viewpoint that the crimped composite fiber is obtained from a composite fiber more excellent in crimpability.

The melting point of the olefin-based polymer (B) is 155°C or less, whereby the difference in melting point from the olefin-based polymer (A) is more easily more than 10°C.

The value of ΔTm is calculated from the difference obtained by determining the melting points of the olefin-based polymer (A) and the olefin-based polymer (B) serving as the part (a) and the part (b), respectively.

The melting point in the disclosure is measured as follows.

1) The olefin-based polymers are each placed in a measurement pan of differential scanning calorimetry analysis (DSC), manufactured by PerkinElmer Co., Ltd., heated from 30°C to 200°C at 10°C/min, retained at 200°C for 10 minutes, and thereafter cooled to 30°C at 10°C/min.

2) Next, the olefin-based polymers are each again heated from 30°C to 200°C at 10°C/min, and the respective melting points are determined from peaks here observed.

<ΔMz/Mw>

The Mz/Mw of the olefin-based polymer (A) is preferably larger than the Mz/Mw of the olefin-based polymer (B) in the disclosure.

Thus, the crimped composite fiber can be obtained from a composite fiber excellent in crimpability.

The absolute value of the difference [Mz/Mw (A) - Mz/Mw (B): ΔMz/Mw] between the Mz/Mw (A) of the olefin-based polymer (A) forming the part (a) and the Mz/Mw (B) of the olefin-based polymer (B) forming the part (b) in the disclosure is preferably from 0.01 to 2.0, more preferably from 0.03 to 1.0, still more preferably from 0.05 to 0.40.

The ΔMz/Mw is 0.01 or more, whereby the crimped composite fiber is obtained from a composite fiber excellent in crimpability. In this regard, the ΔMz/Mw is 2.0 or less, whereby spinnability can be favorably retained.

Herein, the Mz is called Z average molecular weight, is known, and is defined by the following Formula (1).

[Math 1]

$$Mz = \frac{\sum M_i^3 N_i}{\sum M_i^2 N_i}$$

$$(1)$$

In Formula (1), Mi is the molecular weight of the polymer [olefin-based polymer (A) and olefin-based polymer (B): hereinafter, referred to as "olefin-based polymer" in the case of a combination thereof.], and Ni is the molar number of the polymer (olefin-based polymer).

It is generally considered that Mz corresponds to a molecular weight more reflecting a higher molecular weight component in the polymer. Accordingly, Mz/Mw represents a molecular weight distribution more reflecting a higher molecular weight component than Mw/Mn as an index of a common molecular weight distribution. The value thereof has an effect on crimpability of a fiber.

The ∆Mz/Mw is calculated from the absolute value of the difference in Mz/Mw between the olefin-based polymer (A) forming the part (a) and the olefin-based polymer (B) forming the part (b), as determined by GPC analysis.

GPC analysis in the disclosure is performed in the following conditions.

1) Thirty mg of a propylene polymer is completely dissolved in 20 mL of o-dichlorobenzene at 145°C.
2) The solution is filtered with a sintered filter having a pore size of 1.0 $\mu$m, thereby providing a sample.
3) The sample is analyzed with GPC and subjected to conversion in terms of polystyrene (PS), thereby determining the average molecular weight and the molecular weight distribution curve.

The measurement instrument and measurement conditions are as follows.

Measurement apparatus gel permeation chromatograph WATERS ALLIANCE GPC2000 SYSTEM (manufactured by Waters Corporation)

Analysis apparatus data processing software EMPOWER 2 (manufactured by Waters Corporation)

| | |
|---|---|
| Columns | TSKGEL GMH6-HT × 2 + TSKGEL GMH6-HTL × 2 (each having a size of 7.5 mm I.D. × 30 cm, manufactured by Tosoh Corporation) |
| Column temperature | 140°C |
| Mobile phase | o-dichlorobenzene (containing 0.025% butylated hydroxytoluene BHT) |
| Detector | differential refractometer |
| Flow rate | 1 mL/min |
| Concentration of sample | 30 mg/20 mL |
| Amount of injection | 500 $\mu$L |
| Sampling time interval | 1 s |
| Column calibration | monodispersed polystyrene (manufactured by Tosoh Corporation) |
| Molecular weight conversion | PS conversion/standard conversion method |

<MFR Ratio>

The ratio of MFR (hereinafter, also referred to as "MFR ratio") between the MFR of the olefin-based polymer (A) forming the part (a) and the MFR of the olefin-based polymer (B) forming the part (b) in the disclosure is not particularly limited, and is preferably from 0.8 to 1.2.

The MFRs of the olefin-based polymer (A) and the olefin-based polymer (B) in the disclosure are each preferably from 20 g/10 min to 100 g/10 min.

Such MFRs in the disclosure are each determined according to ASTM D1238 at a load of 2,160 g and a temperature of 230°C.

<Mass Ratio Between Part (a) and Part (b)>

The mass ratio [(a):(b)] between the part (a) and the part (b) in the crimped composite fiber in the disclosure is preferably from 10:90 to 60:40.

The mass ratio between the part (a) and the part (b) is within the above range, whereby a composite fiber for use in formation of the crimped composite fiber can be more enhanced in crimpability and the nonwoven laminated body can be more enhanced in flexibility.

The mass ratio [(a):(b)] between the part (a) and the part (b) is more preferably from 10:90 to 50:50, still more preferably from 20:80 to 40:60 from the same viewpoint.

An aspect is preferable in which the crimped composite fiber in the disclosure has at least two regions of the part (a) formed of the olefin-based polymer (A) and the part (b) formed of the olefin-based polymer (B), the mass ratio [(a):(b)] between the part (a) and the part (b) is from 10:90 to 60:40, the olefin-based polymer (A) and the olefin-based polymer (B) are different in at least one of Mz/Mw, melting point, or MFR.

The nonwoven laminated body of the disclosure preferably

has at least two regions of the part (a) formed of the olefin-based polymer (A) and the part (b) formed of the olefin-based polymer (B) in the crimped composite fiber,
has a mass ratio [(a):(b)] between the part (a) and the part (b) of from 10:90 to 60:40, and
satisfies at least one of a condition in which the melting point of the olefin-based polymer (A) is higher than the melting point of the olefin-based polymer (B) or a condition in which the Mz/Mw of the olefin-based polymer (A) is larger than the Mz/Mw of the olefin-based polymer (B).

The crimped composite fiber in the disclosure may be a crimped composite fiber including the olefin-based polymer (A) and the olefin-based polymer (B) and having at least two regions of the part (a) and the part (b) in a transverse section, in which
the mass ratio [(a):(b)] between the part (a) and the part (b) is from 10:90 to 60:40, the part (a) formed of the olefin-based polymer (A) and the part (b) formed of the olefin-based polymer (B), the difference [Mz/Mw (A) - Mz/Mw (B): ΔMz/Mw] between the Mz/Mw (A) of the olefin-based polymer (A) and the Mz/Mw (B) of the olefin-based polymer (B) is from 0.10 to 2.2, and the difference [ΔTm = Tm (A) - Tm (B)] between the melting point [Tm (A)] of the olefin-based polymer (A) and the melting point [Tm (B)] of the olefin-based polymer (B) is more than 10°C.

The crimped composite fiber in the disclosure preferably has an average fiber diameter of 8 μm or more, more preferably 10 μm or more, still more preferably 14 μm or more, from the viewpoint of yarn properties, crimpability, and excellent neck-in resistance and mechanical strength of the nonwoven laminated body in the case of formation of the nonwoven laminated body.

The crimped composite fiber in the disclosure preferably has an average fiber diameter of 23 μm or less, more preferably 20 μm or less, still more preferably 17 μm or less, particularly preferably 16.6 μm or less, from the viewpoint of excellent flexibility of the nonwoven laminated body by itself in the case of formation of the nonwoven laminated body.

The average fiber diameter in the disclosure is measured by a method described in the section of Examples.

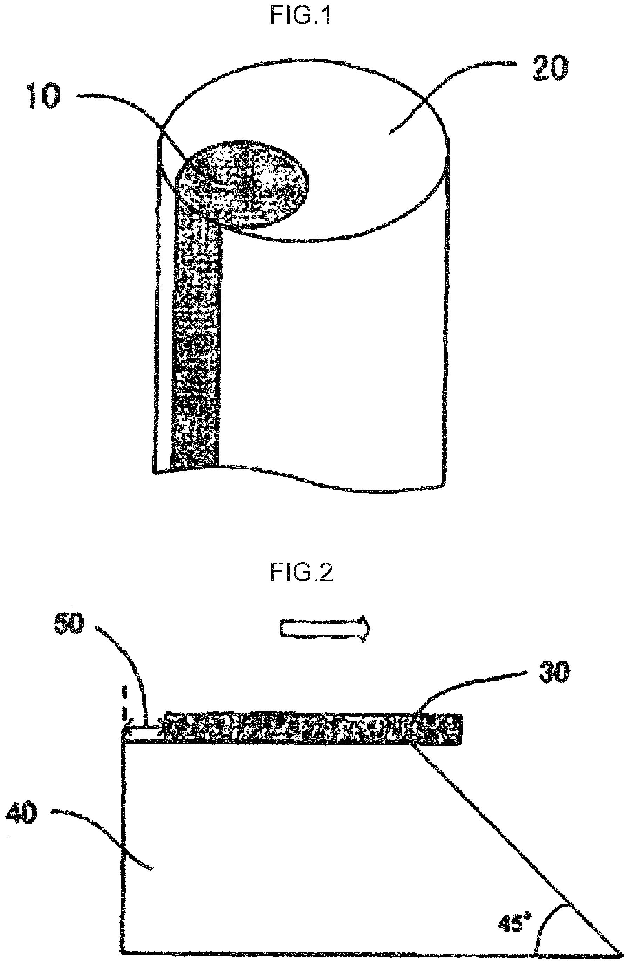

Figure 1 is a perspective view illustrating one example of the crimped composite fiber in the disclosure. In the Figure, reference numeral 10 represents the part (a) and reference numeral 20 represents the part (b).

The crimped composite fiber in the disclosure has at least two regions of the part (a) and the part (b) in its transverse section, and the mass ratio [(a):(b)] of the proportion of the part (a) and the proportion of the part (b) in the transverse section of the crimped composite fiber is from 10:90 to 60:40, preferably from 10:90 to 50:50, more preferably from 20:80 to 40:60, as described above.

The crimped composite fiber having such a configuration is not particularly limited, and can have any of various known shapes. Specific examples can include a side-by-side type (parallel type) crimped composite fiber in which the part (a) and the part (b) are in contact with each other, or a core/sheath type crimped composite fiber in which the part (a) serves as a core part (a') and the part (b) serves as a sheath part (b').

The core/sheath type crimped composite fiber refers to a fiber that includes a core part and a sheath part and that is crimped. The core part (a') refers to a part that is arranged so as to be at least partially surrounded by a polymer different from that of the core part (a') in the cross section of the fiber and that extends in the length direction of the fiber.

The sheath part (b') refers to a part that is arranged so as to at least partially surround the core part (a') in the cross section of the fiber and that extends in the length direction of the fiber. Any core/sheath type crimped composite fiber in which the center of the core part (a') and the center of the sheath part (b'), of the fiber, in the cross section of the fiber are not identical is referred to as "crimped composite fiber having an eccentric core/sheath structure" (also referred to as "eccentric core/sheath type crimped composite fiber".).

Eccentric Core/Sheath Structure

The crimped composite fiber in the nonwoven laminated body of the disclosure preferably has an eccentric core/sheath structure in which the part (a) is the core part (a') and the part (b) is the sheath part (b').

The crimped composite fiber has the eccentric core/sheath structure, whereby the composite fiber for use in formation of the crimped composite fiber can be more improved in crimpability and the nonwoven laminated body can be more enhanced in flexibility.

The eccentric core/sheath structure may be, for example, a structure in which the part (a) formed of the olefin-based polymer (A) larger in Mz/Mw is used in the core part and the part (b) formed of the olefin-based polymer (B) smaller in Mz/Mw is used in the sheath part.

The eccentric core/sheath type crimped composite fiber is present in the form of an "exposure type" fiber in which the side surface of the core part (a') is exposed, or in the form of a "no exposure type" fiber in which the side surface of the core part (a') is not exposed.

The eccentric core/sheath type crimped composite fiber is preferably an exposure type eccentric core/sheath type crimped composite fiber. The reason is because an eccentric core/sheath type crimped composite fiber excellent in crimpability can be obtained. The cross section in which the core part (a') and the sheath part (b') is in contact with each other may be straight or curved, and the cross section of the core part may be round, or elliptic or rectangular.

Any composite fiber in which a bonded surface between the core part and the sheath part is straight and the core part is partially exposed on the surface of the crimped composite fiber is also referred to as a "side-by-side type" fiber.

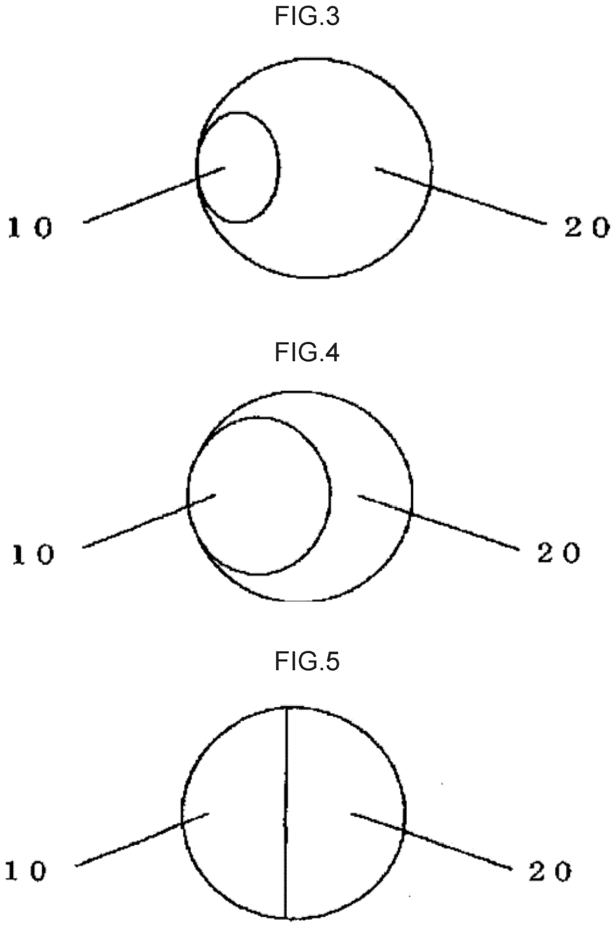

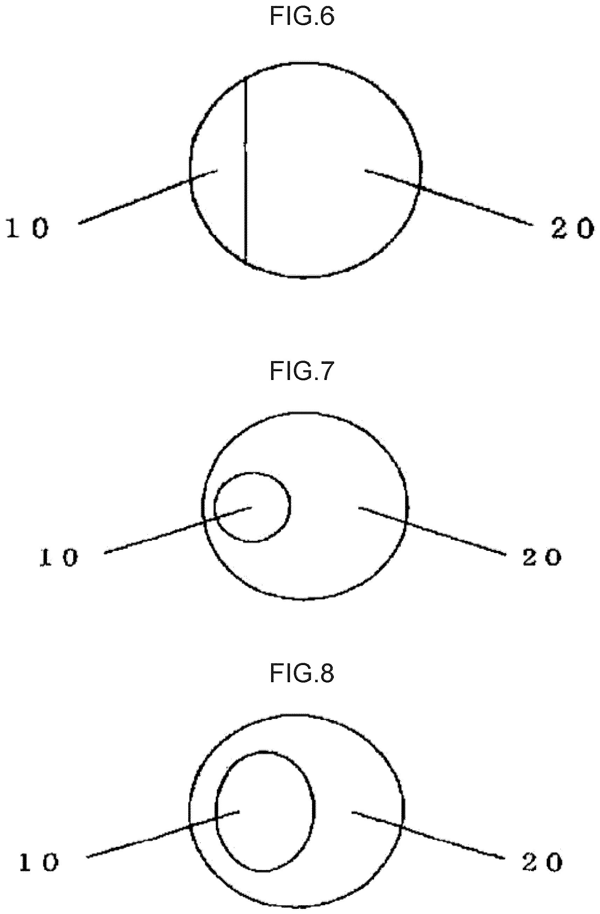

Figure 3 to Figure 8 illustrate other examples of a cross-sectional view of the crimped composite fiber in the disclosure.

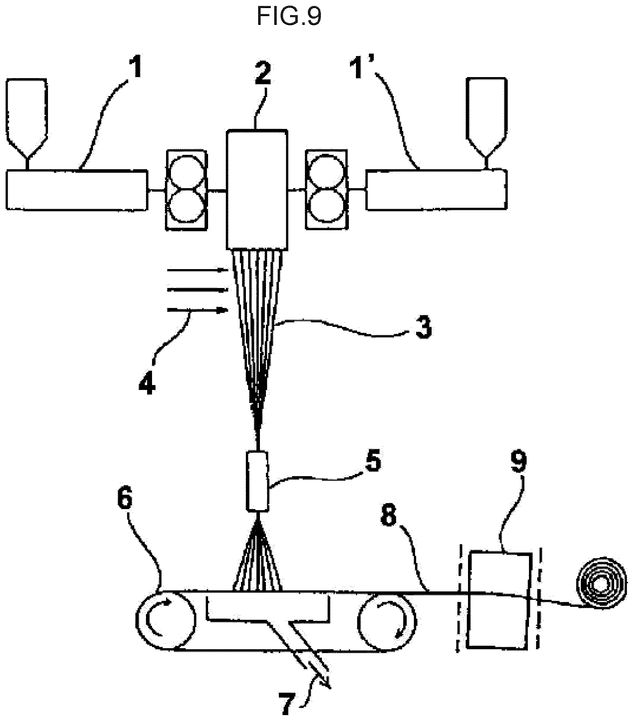

The method of producing the crimped composite fiber, here used, can be any known method. For example, a nonwoven fabric production apparatus illustrated in Figure 9 can form a mode of the crimped composite fiber, for example, the core/sheath type crimped composite fiber, in spinning of raw materials extruded from extruders 1 and 1' that extrude such raw materials.

The crimped composite fiber in the disclosure may be a short fiber or a long fiber, and is preferably a long fiber because, in a case in which a nonwoven fabric is formed, no crimped composite fiber drops from the nonwoven fabric and fluff resistance is excellent. The number of crimps in the crimped composite fiber is not particularly limited, and is, for example, 5 crimps/25 mm or more. The number of crimps is preferably 20 crimps/25 mm or more, more preferably 25 crimps/25 mm or more from the viewpoint that a spunbond nonwoven fabric is obtained which is bulky and excellent in flexibility.

Examples of the nonwoven fabric using the crimped composite fiber can include an actually crimped spunbond nonwoven fabric obtained by crimping with distortion of the fiber upon cooling. Examples of a long fiber in the actually crimped spunbond nonwoven fabric include an eccentric core/sheath type composite long fiber, a parallel type (side-by-side type) composite long fiber, and an eccentric hollow composite long fiber, each obtained by combining heterogeneous thermoplastic resins different in crystallization temperature, melting point, softening point, rate of crystallization, melt viscosity, and/or the like.

<Nonwoven Fabric Layer B Including Extra-Fine Fiber>

The nonwoven laminated body of the disclosure includes a nonwoven fabric layer B including an extra-fine fiber having an average fiber diameter of less than 1.35 μm.

The nonwoven laminated body of the disclosure includes the nonwoven fabric layer B, whereby the nonwoven laminated body can be enhanced in neck-in resistance.

The extra-fine fiber preferably corresponds to a melt blown nonwoven fabric produced by a melt blown method from the viewpoint of a much more enhancement in neck-in properties of the nonwoven laminated body.

The nonwoven fabric layer B may include any of various known nonwoven fabrics such as a melt blown nonwoven fabric, a card-type air-through nonwoven fabric, an air laid nonwoven fabric, a needle-punched spunbond nonwoven fabric, a wet nonwoven fabric, a dry nonwoven fabric, a dry pulp nonwoven fabric, a flash spun nonwoven fabric, and an open nonwoven fabric, other than the extra-fine fiber, and preferably includes only the extra-fine fiber.

The extra-fine fiber in the nonwoven laminated body of the disclosure has an average fiber diameter of less than 1.35 μm.

Thus, a laminated body excellent in uniformity and favorable in texture can be obtained, and a nonwoven laminated body excellent in neck-in resistance can be obtained.

The extra-fine fiber preferably has an average fiber diameter of 1.34 μm or less from the same viewpoint.

The extra-fine fiber preferably has an average fiber diameter of 1.00 μm or more from the viewpoint that a nonwoven laminated body is obtained which is high in single yarn strength and which exhibits sufficient mechanical properties.

The average fiber diameter is more preferably 1.20 μm or more, still more preferably 1.30 μm or more, from the viewpoint that not only a nonwoven laminated body exhibiting sufficient mechanical properties can be obtained, but also a laminated body excellent in uniformity and favorable in texture can be obtained and a nonwoven laminated body excellent in neck-in resistance can be obtained.

The method of measuring the average fiber diameter of the extra-fine fiber is as described in the section of Examples.

The ratio of the basis weight of the nonwoven fabric layer B with respect to the total basis weight of the nonwoven laminated body is from 2.0% to 8.0%.

The ratio of the basis weight of the nonwoven fabric layer B with respect to the total basis weight of the nonwoven laminated body is 2.0% or more, whereby a laminated body excellent in uniformity and favorable in texture can be obtained and a nonwoven laminated body excellent in neck-in resistance can be obtained.

The ratio of the basis weight of the nonwoven fabric layer B with respect to the total basis weight of the nonwoven laminated body is preferably 5.0% or more, more preferably 6.0% or more, still more preferably 6.3% or more, from the same viewpoint.

The ratio of the basis weight of the nonwoven fabric layer B with respect to the total basis weight of the nonwoven laminated body is 8.0% or less, whereby a nonwoven laminated body low in flexural rigidity and excellent in flexibility of such a nonwoven laminated body by itself can be obtained.

The ratio of the basis weight of the nonwoven fabric layer B with respect to the total basis weight of the nonwoven laminated body is preferably 8.0% or less, more preferably 7.0% or less, still more preferably 6.8% or less, from the same viewpoint.

The "coefficient of neck-in" in the disclosure is a value calculated as "width retention rate (%)/basis weight (g/m$^2$)".

The "width retention rate (%)" and the "basis weight (g/m$^2$)" are each a value calculated by a method described in the section of Examples.

The "proportion of the basis weight of the extra-fine fiber in the total basis weight of the laminated body" in the disclosure is a value calculated as "Basis weight (g/m$^2$) of nonwoven fabric layer B/Basis weight (g/m$^2$) of nonwoven laminated body".

The method of measuring the width retention rate is as described in the section of Examples.

(Water Pressure Resistance)

The value of water pressure resistance and the basis weight of the extra-fine fiber are known to be positively correlated in the nonwoven laminated body of the disclosure. Thus, the basis weight of the extra-fine fiber can be estimated from the value of water pressure resistance.

Thus, the basis weight of the extra-fine fiber, estimated from the value of water pressure resistance, can be used to approximately calculate the proportion of the basis weight of the nonwoven fabric layer B.

A conversion formula for estimating the basis weight of the extra-fine fiber from the value of water pressure resistance is represented by Formula 1 below.

Formula 1 is an expression calculated based on the data of an SMS nonwoven laminated body in Experimental Examples in WO 2017/198336 and the data in Experimental Examples in the disclosure.

$$\text{Water pressure resistance [mmH}_2\text{O]} = 55.7 \times \text{Basis weight [g/m}^2\text{] of Extra-fine fiber} + 97.0 \quad \text{Formula 1}$$

The basis weight of the extra-fine fiber, estimated from the value of water pressure resistance by use of the conversion formula, can be used to calculate the ratio of the basis weight of the nonwoven fabric layer B with respect to the total basis weight of the nonwoven laminated body.

Examples of the material included in the extra-fine fiber can include various known thermoplastic resins, for example, an olefin-based polymer such as high-pressure low-density polyethylene, linear low-density polyethylene (so-called LLDPE), high-density polyethylene, a propylene-based polymer (propylene homopolymer, polypropylene random copolymer, propylene/a-olefin copolymer, or the like), poly1-butene, poly(4-methyl-1-pentene), an ethylene/propylene random copolymer, an ethylene/1-butene random copolymer, or a propylene/1-butene random copolymer, as a homopolymer or a copolymer of any α-olefin such as ethylene, propylene, 1-butene, 1-pentene, 1-hexene, 1-octene, 1-decene, 3-methyl-1-butene, 3-methyl-1-pentene, 3-ethyl-1-pentene, 4-methyl-1-pentene, and/or 4-methyl-1-hexene; polyester such as polyethylene terephthalate, polybutylene terephthalate, or polyethylene naphthalate; a polyamide-based polymer such as nylon-6, nylon-66, or poly(meta-xylene adipamide); polyvinyl chloride, polyimide, an ethylene/vinyl acetate copolymer, polyacrylonitrile, polycarbonate, polystyrene, an ionomer, thermoplastic polyurethane, or any mixture thereof.

In particular, the material included in the extra-fine fiber is preferably an olefin-based polymer, more preferably a propylene-based polymer.

<Propylene-Based Polymer>

The extra-fine fiber is preferably any propylene-based polymer as the olefin-based polymer, from the viewpoint of chemical resistance.

Such any propylene-based polymer is preferably a homopolymer of propylene, having a melting point (Tm) of 155°C or more, preferably in a range of from 157°C to 165°C, or a propylene/a-olefin copolymer of propylene and one or more α-olefins (excluding propylene) each having 2 or more carbon atoms.

Such one or more α-olefins (excluding propylene) each having 2 or more carbon atoms are preferably one or more α-olefins (for example, ethylene, 1-butene, 1-pentene, 1-hexene, 1-octene, 1-decene, 3-methyl-1-butene, 3-methyl-1-pentene, 3-ethyl-1-pentene, 4-methyl-1-pentene, and/or 4-methyl-1-hexene) each having from 2 to 10 carbon atoms, more preferably one or more α-olefins each having from 2 to 8 carbon atoms. In particular, a propylene homopolymer is more preferable. The content of a structural unit derived from the α-olefin of the propylene/a-olefin copolymer is preferably from 0.5% by mass to 25% by mass, more preferably from 1.0% by mass to 20% by mass with respect to the entire structural unit.

The propylene-based polymer is not particularly limited in terms of the melt flow rate (MFR: ASTM D 1238, 230°C, load 2,160 g) as long as the polymer can be melt spun, and may be, for example, from 1 g/10 min to 3,000 g/10 min, and is preferably from 500 g/10 min to 2,000 g/10 min, still more preferably from 700 g/10 min to 1,600 g/10 min.

The basis weight of the nonwoven fabric layer B in the nonwoven laminated body of the disclosure is preferably in a range of from 0.5 g/m$^2$ to 1.5 g/m$^2$, more preferably in a range of from 0.7 g/m$^2$ to 1.5 g/m$^2$. The basis weight of the nonwoven fabric layer B is from 0.5 g/m$^2$ to 1.5 g/m$^2$, whereby a thin crimped nonwoven fabric is obtained which exhibits neck-in resistance and flexibility in a well-balanced manner.

The method of measuring the basis weight in the disclosure is as described in the section of Examples.

(Coefficient of Neck-In)

The nonwoven laminated body of the disclosure has a coefficient of neck-in of 5.0 or more, in a case in which the nonwoven laminated body is expanded at 100 N/m.

The nonwoven laminated body of the disclosure has a coefficient of neck-in of 5.0 or more, whereby the basis weight of the nonwoven laminated body can be suppressed and a neck-in phenomenon can be favorably suppressed.

The nonwoven laminated body preferably has a coefficient of neck-in of 5.2 or more, more preferably 5.4 or more, from the same viewpoint, in a case in which the nonwoven laminated body is expanded at 100 N/m.

Examples of the method of adjusting the coefficient of neck-in include the following methods.

A method of adjusting the coefficient of neck-in by selecting a resin having an appropriate unit structure and an appropriate binding pattern of the unit structure, as each resin included in the crimped composite fiber and the extra-fine fiber.

A method of adjusting the coefficient of neck-in by selecting a resin having appropriate physical properties (for example, molecular weight distribution and/or crystallinity), as each resin included in the crimped composite fiber and the extra-fine fiber.

A method of adjusting the coefficient of neck-in by modifying the proportion of the basis weight of each layer in the laminated body.

Preferably, the nonwoven fabric layer A is a spunbond nonwoven fabric layer and the nonwoven fabric layer B is a melt blown nonwoven fabric layer in the nonwoven laminated body of the disclosure.

(Layer Including Other Fiber)

The nonwoven laminated body of the disclosure may include any layer including other fiber, other than the nonwoven fabric layer A and the nonwoven fabric layer B.

Examples of such any layer including other fiber can include respective layers including various known nonwoven fabrics, such as a layer including a spunbond nonwoven fabric, not corresponding to the nonwoven fabric layer A, a layer including a melt blown nonwoven fabric, not corresponding to the nonwoven fabric layer B, a layer including a wet nonwoven fabric, a layer including a dry nonwoven fabric, a layer including a dry pulp nonwoven fabric, a layer including a flash spun nonwoven fabric, and a layer including am open nonwoven fabric.

In particular, such any layer including other fiber is preferably a layer including a melt blown nonwoven fabric, not corresponding to the nonwoven fabric layer B, from the viewpoint that not only neck-in resistance is enhanced, but also cushioning properties and flexibility demanded for recent sanitary materials are kept at high levels.

(Basis Weight of Nonwoven Laminated Body)

The basis weight (mass per unit area of the nonwoven laminated body) of the nonwoven laminated body of the disclosure is preferably from 8 $g/m^2$ to 25 $g/m^2$, more preferably from 10 $g/m^2$ to 23 $g/m^2$, still more preferably from 12 $g/m^2$ to 18 $g/m^2$, particularly preferably from 12 $g/m^2$ to 15 $g/m^2$, from the viewpoint of suppression of a neck-in phenomenon.

The nonwoven laminated body of the disclosure is preferably heat-fused by embossing.

The crimped composite fiber in the nonwoven laminated body is mutually heat-fused by embossing, whereby stability and strength of the fiber can be favorably maintained.
The nonwoven laminated body of the disclosure preferably has an embossing area ratio of from 5% to 20%, by the embossing. The range enables both mechanical strength and flexibility demanded for a sanitary material to be achieved.
The embossing area ratio by the embossing is more preferably from 7% to 15% from the same viewpoint.
The method of the embossing, here used, can be any known method, and may be, for example, a method described in Japanese Patent Application Laid-Open (JP-A) No. 2017-153901.

<Application or the like of Nonwoven Laminated Body>

The nonwoven laminated body of the disclosure can be stacked on various layers and thus applied to various applications.
Specific examples can include a knitted fabric, a woven fabric, a nonwoven fabric, a film, and a sanitary product. In particular, the nonwoven laminated body is preferably applied to a sanitary product including the nonwoven laminated body of the disclosure because the nonwoven laminated body of the disclosure exhibits favorable cushioning properties, flexibility, and neck-in resistance.
In a case in which the nonwoven fabric layer A including the crimped composite fiber, the nonwoven fabric layer B including the extra-fine fiber, and, if necessary, such any layer including other fiber are stacked (attached) in the disclosure, any of various known methods can be adopted, for example, embossing, a heat-fusing method such as ultrasonic wave fusion, a mechanical interlacing method such as needle punch or water jet, a method with an adhesive such as a hot melt adhesive or a urethane-based adhesive, and extrusion lamination.

(Aspect of Nonwoven Laminated Body)

A preferable aspect of the nonwoven laminated body of the disclosure is, for example, a laminated body of a spunbond nonwoven fabric (crimped) including a crimped composite fiber produced by a spunbond method and a melt blown nonwoven fabric (extra-fine fiber) including a melt blown fiber, and any other nonwoven fabric stacked, if necessary.
Specific examples include

a laminated body having a four-layer structure of spunbond nonwoven fabric (crimped)/melt blown nonwoven fabric (extra-fine fiber)/spunbond nonwoven fabric (crimped)/spunbond nonwoven fabric (crimped), spunbond nonwoven fabric (not crimped)/melt blown nonwoven fabric (extra-fine fiber)/spunbond nonwoven fabric (crimped)/spunbond nonwoven fabric (crimped), or spunbond nonwoven fabric (not crimped)/melt blown nonwoven fabric (extra-fine fiber)/spunbond nonwoven fabric (not crimped)/spunbond nonwoven fabric (crimped);
a laminated body having a three-layer structure of spunbond nonwoven fabric (crimped)/melt blown nonwoven fabric (extra-fine fiber)/spunbond nonwoven fabric (crimped) or spunbond nonwoven fabric (not crimped)/melt blown nonwoven fabric (extra-fine fiber)/spunbond nonwoven fabric (crimped); and
a laminated body having a two-layer structure of melt blown nonwoven fabric (extra-fine fiber)/spunbond nonwoven fabric (crimped).

In particular, the nonwoven laminated body of the disclosure is preferably a laminated body of the three-layer structure and the four-layer structure, more preferably a laminated body of the four-layer structure, from the viewpoint that the nonwoven laminated body achieves both flexibility and neck-in resistance.
In particular, the nonwoven laminated body of the disclosure still more preferably has a structure of spunbond nonwoven fabric (crimped)/melt blown nonwoven fabric (extra-fine fiber)/spunbond nonwoven fabric (crimped)/spunbond nonwoven fabric (crimped), from the viewpoint that the nonwoven laminated body achieves both flexibility and neck-in resistance.

(Method of Producing Nonwoven Laminated Body)

The method of producing the nonwoven laminated body is not particularly limited, and any known method can be used.

For example, a nonwoven fabric production apparatus illustrated in Figure 9 can be used.

The nonwoven fabric production apparatus includes respective extruders 1 and 1' that extrude the olefin-based polymer (A) and the olefin-based polymer (B), a spinneret 2 that spins such each raw material extruded, thereby forming a fiber 3, an ejector 5 that stretches the fiber 3, a collector apparatus 6 that collects the fiber stretched, thereby forming a nonwoven fabric 8, a bonding unit 9 that heat-fuses at least a portion of the nonwoven fabric 8, and a winder that winds up the nonwoven fabric heat-fused. The collector apparatus 6 includes a suction unit 7 that efficiently collects the fiber by suction, on the lower portion of a surface (collecting surface) for collection of the fiber. The fiber 3 obtained by the spinneret 2 is cooled by air (quench air) that cools the fiber, in the nonwoven fabric production apparatus.

Examples

Hereinafter, the disclosure will be still more specifically described with reference to Examples, but the disclosure is not limited to the following Examples departing from the gist thereof. Unless particularly noted, "part(s)" is on a mass basis.

The basis weight ($g/m^2$) in the Examples was measured by the following method.

Ten test pieces of 100 mm (fiber flow direction: MD) $\times$ 100 mm (direction perpendicular to the fiber flow direction: CD) were taken from a nonwoven fabric to be measured. The locations at which such test pieces were taken were ten locations in the CD direction. Next, the mass [g] of each of such test pieces taken was measured with an electron even balance (manufactured by KENSEI KOGYO Co., Ltd.), and the average value of the mass with respect to the test pieces was determined.

The average value determined above was converted to the mass [g] per square meter, and the resultant was defined as the basis weight [$g/m^2$] of each nonwoven fabric.

The average fiber diameter in Examples was measured by the following method.

First, each fiber nonwoven fabric was photographed at a magnification appropriately adjusted (for example, 200x), with an electron microscope, any fifty fibers among fibers included in such each fiber nonwoven fabric were selected, and the width (diameter) of each of the fibers selected was measured. The average value of the width (diameter) with respect to the fibers measured, was calculated, and was defined as the average fiber diameter.

The average fiber diameter of the extra-fine fiber (including the melt blown fiber) was also determined by the above method of measuring the average fiber diameter at a magnification appropriately adjusted (for example, 1,000x), with the electron microscope.

The width retention rate was calculated by the following method.

First, both ends of a nonwoven laminated body of 200 mm in width $\times$ 450 mm in length, in the length direction, were clamped by respective tools at a distance between such tools used, of 350 mm. Next, the nonwoven laminated body was installed to a tensile tester and pulled at a tension of 100 N/m, and the width was then measured. The width retention rate was calculated by the following Formula.

$$\text{Width retention rate (\%)} = (\text{Width (mm) upon pulling/Initial width (mm)}) \times 100$$

<Evaluation>

The following evaluations were performed with respect to each of Examples and Comparative Examples. The evaluation results are shown in Table 2.

(Tensile Strength)

Each nonwoven laminated body was subjected to measurement according to JIS L 1906. A test piece of 25 mm in width $\times$ 200 mm in length was taken from such each nonwoven laminated body, and subjected to measurement with a tensile tester at five points in MD (fiber flow direction) and at five points in CD (direction perpendicular to the fiber flow direction) at a distance between chucks of 100 mm and a head speed of 100 mm/min, and the respective average values were calculated, thereby determining the tensile strength (N/25 mm).

(Water Pressure Resistance)

The water pressure resistance was measured according to a JIS L1072A method (low water pressure method). Four test pieces of about 15 × 15 cm were each taken, and installed to a water resistance testing apparatus (manufactured by TESTER SANGYO CO., LTD.) so that the surface of such each test piece was touched with water, water pressure was applied to such each test piece by uplifting a level apparatus filled with water at normal temperature, at a rate of 60 ± 3 cm/min or 10 ± 0.5 cm/min, the water level at which water was leaked through opposite three points in such each test piece was measured, and the pressure here was used to determine the water pressure resistance.

(Flexibility)

The flexibility was evaluated by a so-called cantilever method according to JIS L1096. Specifically, the following method was performed.

1) A test piece 30 of 2 cm × 15 cm was prepared, and left to still stand on a testing stand 40 illustrated in Figure 2.
2) The test piece 30 was slowly extruded in a direction indicated by an arrow, and a distance 50, at which the test piece was moved until bending, was measured.
3) Such measurement was made in a case in which the MD of the test piece was parallel to the movement direction and in a case in which the CD of the test piece was parallel to the movement direction.

The nonwoven laminated body was more excellent in rigidity, as the numerical value obtained by the measurement was higher, and the nonwoven laminated body was more excellent in flexibility, as the numerical value was lower.

(Coefficient of Neck-In)

The coefficient of neck-in, as the index of neck-in resistance, was calculated as a value calculated as width retention rate (%)/basis weight (g/m$^2$), by use of the width retention rate which was measured according to any existing method.

The neck-in resistance was more favorable, as the coefficient of neck-in was higher.

(Spinnability)

The number of broken yarns was measured in spinning of the polymer included in each layer in Examples or Comparative Examples, and was evaluated according to the following criteria.

A: no broken yarns
B: any broken yarn was caused one or more times.

(Moldability)

An embossing roll was travelled in embossing for 5 minutes, and any attachment state occurring upon passing of each nonwoven laminated body through the embossing roll was confirmed, and was evaluated according to the following criteria.

A: a state in which no attachment was visually confirmed at all.
B: a state in which any attachment was visually confirmed or a state in which the nonwoven laminated body was wound on the embossing roll

<Example 1>

(First Layer)

A first layer was produced by performing combined melt spinning according to a spunbond method by use of the following component IA and the following IB component, and depositing a nonwoven web formed from a side-by-side type crimped composite fiber (hereinafter, designated as "crimped composite fiber I") on a movement/collection plane. The mass ratio of component IA/component IB in the crimped composite fiber I was 40/60.

Table 1 shows the average fiber diameter of the crimped composite fiber I in the first layer and the basis weight in the first layer.

-Olefin-based polymer (A) (component IA) in crimped composite fiber I-

A propylene homopolymer (h-PP) having a melting point of 162°C, a Mz/Mw of 2.24, and a MFR pf 60 g/10 min (measured according to ASTM D1238 at a temperature of 230°C and a load of 2,160 g, and the same applied to the following, unless particularly specified.)

-Olefin-based polymer (B) (component IB) in crimped composite fiber I-

A propylene/ethylene random copolymer (r-PP) having a melting point of 142°C, a Mz/Mw of 2.19, and a MFR of 60 g/10 min

(Second Layer)

A second layer was produced on the nonwoven web of the first layer according to the same method and components as the method and components described with respect to the first layer, and thus a nonwoven web having a stacked structure was produced.
Table 1 shows the average fiber diameter of the crimped composite fiber I in the second layer and the basis weight in the second layer.

(Third Layer)

A third layer was produced by feeding a propylene homopolymer having a melting point of 159°C and a MFR of 850 g/10 min, to a die of a melt blown nonwoven fabric production apparatus, discharging the homopolymer from the die heated to a molding temperature described in Table 1, by use of a nozzle for melt blowing (diameter 0.32 mm, 41 holes/inch), together with high-temperature and high-pressure air at a molding temperature described in Table 1, blown out through both ends of the nozzle, and in-line depositing a fiber (non-crimped composite fiber I) discharged at DCD (distance from the surface of a spinneret to a collector) described in Table 1, on the nonwoven web of the first layer and the second layer, and thus a nonwoven web having a stacked structure was produced.
Table 1 shows the average fiber diameter of the non-crimped composite fiber I in the third layer and the basis weight in the third layer.

(Fourth Layer)

A fourth layer was produced on the nonwoven web of the third layer according to the same method and components as the method and components described with respect to the first layer, and thus a nonwoven web having a stacked structure was produced.
Table 1 shows the average fiber diameter of the crimped composite fiber I in the fourth layer and the basis weight in the fourth layer.
Next, the nonwoven web having a stacked structure was heat-fused by the following embossing roll in the following embossing conditions, and thus a SSMS nonwoven laminated body was obtained. The embossing area ratio of a pressure-bonded part was 11%. Table 1 shows the total basis weight of the entire nonwoven laminated body.

-Embossing roll-

Embossing area ratio: 11%
Aspect ratio of embossing: 4.1 mm/mm$^2$
Rockwell hardness of embossing material: 37 HRC

-Embossing conditions -

Embossing temperature: 142°C
Embossing linear pressure: 784 N/cm

<Example 2, Example 3, Comparative Example 1, and Comparative Example 2>

Each SSMS nonwoven laminated body having a total basis weight described in Table 1 was obtained in the same manner as in Example 1 except that the average fiber diameter with respect to the first layer, the second layer, and the fourth layer was adjusted as described in Table 1, the average fiber diameter with respect to the third layer was adjusted as described in Table 1, and the basis weights of the first layer to the fourth layer were changed as described in Table 1.

<Comparative Example 3>

(First Layer)

A first layer was produced according to the same method as the method described in the section (First Layer) in Example 1.

Table 1 shows the average fiber diameter of the crimped composite fiber I in the first layer and the basis weight in the first layer.

(Second Layer)

A second layer was produced by performing combined melt spinning according to a spunbond method by use of the following component IIA and the following component IIB, and in-line depositing a nonwoven web formed from a side-by-side type crimped composite fiber (hereinafter, designated as "crimped composite fiber II"), on the nonwoven web of the first layer, and thus a nonwoven web having a stacked structure was produced. The mass ratio of component IIA/component IIB in the crimped composite fiber II was 40/60.

Table 1 shows the average fiber diameter of the crimped composite fiber II in the second layer and the basis weight in the second layer.

-Olefin-based polymer (A) (component IIA) of crimped composite fiber II-

A mixed product (Mz/Mw 2.54) of a propylene/ethylene random copolymer having a melting point of 142°C and a MFR of 60 g/10 min and a propylene homopolymer having a melting point of 162°C and a MFR of 3 g/10 min at a mass ratio (propylene/ethylene random copolymer/propylene homopolymer) of 96:4.

-Olefin-based polymer (B) (component IIB) of crimped composite fiber II-

A propylene/ethylene random copolymer having a melting point of 142°C, a Mz/Mw of 2.19, and a MFR of 60 g/10 min

(Third Layer)

A third layer was produced according to the same method as the method described in the section (Third layer) in Example 1 except that the molding temperature was a temperature described in Table 1 and DCD was DCD described in Table 1.

Table 1 shows the average fiber diameter of the non-crimped composite fiber I in the third layer and the basis weight in the third layer.

(Fourth Layer)

A fourth layer was produced on the nonwoven web of the third layer according to the same method and components as the method and components described with respect to the second layer, and thus a nonwoven web having a stacked structure was produced.

Table 1 shows the average fiber diameter of the crimped composite fiber II in the fourth layer, and the basis weight in the fourth layer.

Next, the nonwoven web having a stacked structure was heat-fused by an embossing roll according to the same method and embossing conditions as those in Example 1, and thus an SSMS nonwoven laminated body was obtained. The embossing area ratio of a pressure-bonded part was 11%. Table 1 shows the total basis weight of the entire nonwoven laminated body.

<Comparative Example 4 and Comparative Example 5>

Each SSMS nonwoven laminated body having a total basis weight described in Table 1 was obtained in the same manner as in Comparative Example 3 except that the average fiber diameter with respect to the first layer, the second layer, and the fourth layer was adjusted as described in Table 1, the average fiber diameter with respect to the third layer was adjusted as described in Table 1, and the basis weights of the first layer to the fourth layer were changed as described in Table 1.

<Comparative Example 6 and Comparative Example 7>

Each nonwoven laminated body having a total basis weight described in Table 1 was obtained in the same manner as in Comparative Example 3 except that the average fiber diameter with respect to the first layer, the second layer, and the fourth layer was adjusted as described in Table 1, the basis weight of the first layer, the second layer, and the fourth layer were changed as described in Table 1, and no third layer was produced.

[Table 1]

| | | Example 1 | Example 2 | Example 3 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|
| Fourth layer (SB) | Raw material | Crimped fiber I | Crimped fiber I | Crimped fiber I | Crimped fiber I | Crimped fiber I | Crimped fiber II | Crimped fiber II | Crimped fiber II | Crimped fiber II | Crimped fiber II |
| Third layer (MB) | Raw material | Non-crimped I | Non-crimped I | Non-crimped I | Non-crimped I | Non-crimped I | Non-crimped I | Non-crimped I | Non-crimped I | - | - |
| | Molding temperature [°C] | 285 | 285 | 285 | 285 | 285 | 270 | 270 | 270 | - | - |
| | DCD [mm] | 115 | 115 | 115 | 115 | 115 | 130 | 130 | 130 | - | - |
| Second layer (SB) | Raw material | Crimped fiber I | Crimped fiber I | Crimped fiber I | Crimped fiber I | Crimped fiber I | Crimped fiber II | Crimped fiber II | Crimped fiber II | Crimped fiber II | Crimped fiber II |
| First layer (SB) | Raw material | Crimped fiber I | Crimped fiber I | Crimped fiber I | Crimped fiber I | Crimped fiber I | Crimped fiber I | Crimped fiber I | Crimped fiber I | Crimped fiber I | Crimped fiber I |
| Average fiber diameter (μm) with respect to first, second, and fourth layers (SB) | | 15.8 | 16.5 | 15.1 | 15.5 | 15.9 | 15.3 | 15.3 | 15.3 | 14.8 | - |
| Average fiber diameter (μm) with respect to third layer (MB) | | 1.34 | 1.30 | 1.32 | 1.34 | 1.33 | 1.68 | 1.44 | 1.65 | - | - |
| Basis weight (g/m²) | Total basis weight | 13 | 13 | 13 | 13 | 13 | 17 | 17 | 17 | 17 | 13 |
| | Fourth layer | 4.05 | 4.00 | 4.10 | 3.83 | 4.27 | 5.43 | 5.33 | 5.27 | 5.67 | 4.33 |
| | Third layer | 0.85 | 1.00 | 0.70 | 1.50 | 0.20 | 0.70 | 1.00 | 1.20 | 0.00 | 0.00 |
| | Second layer | 4.05 | 4.00 | 4.10 | 3.83 | 4.27 | 5.43 | 5.33 | 5.27 | 5.67 | 4.33 |
| | First layer | 4.05 | 4.00 | 4.10 | 3.83 | 4.27 | 5.43 | 5.33 | 5.27 | 5.67 | 4.33 |

(continued)

| | Example 1 | Example 2 | Example 3 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|
| Proportion (%) of basis weight of MB | 6.5% | 7.7% | 5.4% | 11.5% | 1.5% | 4.1% | 5.9% | 7.1 % | - | - |

[Table 2]

| | | Example 1 | Example 2 | Example 3 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|
| Strength (N/25 mm) | MD direction | 5.5 | 5.6 | 5.4 | 5.8 | 5.2 | 9.5 | 7.9 | 9.5 | 7.7 | - |
| | CD direction | 1.9 | 2.0 | 1.8 | 2.5 | 1.3 | 4.0 | 3.9 | 4.3 | 3.6 | - |
| Water pressure resistance (mmH$_2$O) | | 125 | 135 | 115 | 170 | 80 | 122 | 160 | 175 | 107 | - |
| Cantilever (mm) | MD direction | 23 | 25 | 21 | 32 | 14 | 29 | 23 | 29 | 21 | - |
| | CD direction | 14 | 14 | 14 | 16 | 12 | 18 | 18 | 18 | 15 | - |
| Width retention rate (%) | | 71.9 | 73.5 | 70.3 | 79 | 65 | 79.4 | 83.3 | 83.5 | 72 | - |
| Coefficient of neck-in | | 5.53 | 5.66 | 5.41 | 6.07 | 4.99 | 4.67 | 4.90 | 4.91 | 4.24 | - |
| Spinnability | | A | A | A | A | A | A | A | A | A | A |
| Moldability | | A | A | A | A | A | A | A | A | A | B |

As described in Table 2, Example 1 to Example 3, in which the nonwoven fabric layer A (each of first, second and fourth layers) including the crimped composite fiber and the nonwoven fabric layer B (third layer) including the extra-fine fiber having an average fiber diameter of less than 1.35 $\mu$m were included and the ratio of the basis weight of the nonwoven fabric layer B with respect to the total basis weight of the nonwoven laminated body was from 2.0% to 8.0%, each exhibited excellent flexibility and neck-in resistance.

Comparative Example 1, in which the ratio of the basis weight of the nonwoven fabric layer B (third layer) with respect to the total basis weight of the nonwoven laminated body was more than 8.0%, exhibited a high value of cantilever and inferior flexibility.

Comparative Example 2, in which the ratio of the basis weight of the nonwoven fabric layer B (third layer) with respect to the total basis weight of the nonwoven laminated body was less than 2.0%, exhibited a low value of coefficient of neck-in and inferior neck-in resistance.

Comparative Example 3 to Comparative Example 5 each had an average fiber diameter of the extra-fine fiber in the nonwoven fabric layer B, of 1.35 $\mu$m or more. Comparative Examples 3 and 5 exhibited inferior flexibility and neck-in resistance, and Comparative Example 4 exhibited inferior neck-in resistance.

Comparative Example 6 and Comparative Example 7 did not include any nonwoven fabric layer B including an extra-fine fiber having an average fiber diameter of less than 1.35 $\mu$m. Comparative Example 6 exhibited inferior neck-in resistance and Comparative Example 7 did not enable any nonwoven laminated body to be collected.

The disclosure of Japanese Patent Application No. 2019-061595 filed on March 27, 2019 is herein incorporated by reference in its entirety.

All documents, patent applications, and technical standards described herein are herein incorporated by reference, as if each individual document, patent application, and technical standard were specifically and individually indicated to be incorporated by reference.

**Claims**

1. A nonwoven laminated body comprising:

   a nonwoven fabric layer A comprising a crimped composite fiber; and
   a nonwoven fabric layer B comprising an extra-fine fiber having an average fiber diameter of less than 1.35 $\mu$m, wherein a ratio of a basis weight of the nonwoven fabric layer B with respect to a total basis weight of the nonwoven laminated body is from 2.0% to 8.0%.

2. The nonwoven laminated body according to claim 1, wherein the extra-fine fiber has an average fiber diameter of 1.00 $\mu$m or more and less than 1.35 $\mu$m.

3. The nonwoven laminated body according to claim 1 or 2, wherein the ratio of the basis weight of the nonwoven fabric layer B with respect to the total basis weight of the nonwoven laminated body is from 5.0% to 8.0%.

4. The nonwoven laminated body according to any one of claims 1 to 3, wherein:

   the nonwoven fabric layer A is a spunbond nonwoven fabric layer, and
   the nonwoven fabric layer B is a melt blown nonwoven fabric layer.

5. The nonwoven laminated body according to any one of claims 1 to 4, wherein:

   the crimped composite fiber has at least two regions including a part (a) formed of an olefin-based polymer (A) and a part (b) formed of an olefin-based polymer (B),
   a mass ratio [(a):(b)] between the part (a) and the part (b) is from 10:90 to 60:40, and
   at least one of a condition in which a melting point of the olefin-based polymer (A) is higher than a melting point of the olefin-based polymer (B) or a condition in which Mz/Mw of the olefin-based polymer (A) is larger than the Mz/Mw of the olefin-based polymer (B) is satisfied.

6. The nonwoven laminated body according to claim 5, wherein the olefin-based polymer (A) and the olefin-based polymer (B) are each a propylene-based polymer.

7. The nonwoven laminated body according to claim 5 or 6, wherein the crimped composite fiber has an eccentric core/sheath structure in which the part (a) is a core part (a') and the part (b) is a sheath part (b').

**8.** The nonwoven laminated body according to any one of claims 1 to 7, wherein the nonwoven laminated body is heat-fused by embossing.

**9.** The nonwoven laminated body according to claim 8, wherein an embossing area ratio by the embossing is from 5% to 20%.

**10.** A sanitary product comprising the nonwoven laminated body according to any one of claims 1 to 9.

# FIG.1

# FIG.2

FIG.3

10          20

FIG.4

10          20

FIG.5

10          20

## FIG.6

## FIG.7

## FIG.8

# FIG.9

## INTERNATIONAL SEARCH REPORT

| International application No. |
|---|
| PCT/JP2020/013446 |

| A. CLASSIFICATION OF SUBJECT MATTER |
|---|
| B32B 5/26(2006.01)i; D04H 3/007(2012.01)i; D04H 3/016(2012.01)i; D04H 3/147(2012.01)i; D04H 3/16(2006.01)i; A61F 13/51(2006.01)i<br>FI: B32B5/26; D04H3/007; D04H3/016; D04H3/147; D04H3/16; A61F13/51<br>According to International Patent Classification (IPC) or to both national classification and IPC |

| B. FIELDS SEARCHED |
|---|
| Minimum documentation searched (classification system followed by classification symbols)<br>B32B1/00-43/00; D04H1/00-18/04; A61F13/15-13/84; A61L15/16-15/64 |

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | WO 2017/198336 A1 (FIBERTEX PERSONAL CARE A/S) 23.11.2017 (2017-11-23) claims 1, 10, page 2, lines 13-14, page 5, lines 14-17, page 6, lines 1-4, example 6 | 1-4, 10<br>5-10 |
| Y | WO 2011/129211 A1 (MITSUI CHEMICALS, INC.) 20.10.2011 (2011-10-20) claims 1, 2, 4, paragraphs [0009]-[0011], [0104] | 5-10 |
| Y | WO 2012/111723 A1 (MITSUI CHEMICALS, INC.) 23.08.2012 (2012-08-23) claims 1, 5, 9, paragraph [0052], examples | 8-10 |
| A | WO 2017/150728 A1 (MITSUI CHEMICALS, INC.) 08.09.2017 (2017-09-08) claims 1, 3-5, 9, 10, example 5 | 1-10 |
| A | JP 2004-3096 A (ASAHI KASEI CORPORATION) 08.01.2004 (2004-01-08) paragraph [0001], example 21 | 1-10 |

☐ Further documents are listed in the continuation of Box C.    ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 03 June 2020 (03.06.2020) | 16 June 2020 (16.06.2020) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**INTERNATIONAL SEARCH REPORT**
Information on patent family members

| International application No. |
| --- |
| PCT/JP2020/013446 |

| Patent Documents referred in the Report | Publication Date | Patent Family | Publication Date |
| --- | --- | --- | --- |
| WO 2017/198336 A1 | 23 Nov. 2017 | EP 3246443 A1<br>CN 109072513 A<br>KR 10-2019-0008544 A<br>JP 2019-516875 A | |
| WO 2011/129211 A1 | 20 Oct. 2011 | US 2013/0029555 A1<br>claims 1, 2, 4,<br>paragraphs [0010]-<br>[0016], [0148]<br>EP 2559793 A1<br>CN 102844480 A<br>KR 10-2012-0128696 A | |
| WO 2012/111723 A1 | 23 Aug. 2012 | US 2013/0317469 A1<br>claims 1, 4, 5, 9,<br>paragraphs [0063],<br>[0064], examples<br>EP 2677074 A1<br>CN 103370464 A<br>KR 10-2013-0115374 A | |
| WO 2017/150728 A1 | 08 Sep. 2017 | US 2019/0053960 A1<br>claims 1, 3-5, 9, 10,<br>example 5<br>EP 3424475 A1<br>CN 108697562 A<br>KR 10-2018-0108790 A | |
| JP 2004-3096 A | 08 Jan. 2004 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011129211 A
- US 20170335498 A
- WO 2017198336 A
- JP 2017153901 A
- JP 2019061595 A